(19) Europäisches Patentamt
European Patent Office
Office européen des brevets

(11) Veröffentlichungsnummer: **0 235 376**
**A2**

(12) # EUROPÄISCHE PATENTANMELDUNG

(21) Anmeldenummer: **86116930.8**

(51) Int. Cl.4: **C07C 149/243**

(22) Anmeldetag: **05.12.86**

(30) Priorität: **05.03.86 DE 3607167**

(43) Veröffentlichungstag der Anmeldung:
**09.09.87 Patentblatt 87/37**

(84) Benannte Vertragsstaaten:
**BE CH DE FR GB IT LI**

(71) Anmelder: **Degussa Aktiengesellschaft**
**Weissfrauenstrasse 9**
**D-6000 Frankfurt am Main 1(DE)**

(72) Erfinder: **Koban, Hans-Günter, Dr.**
**Kapellenweg 6**
**D-6460 Gelnhausen(DE)**
Erfinder: **Koberstein, Edgar, Dr.**
**Wolfskernstrasse 8**
**D-8755 Alzenau(DE)**
Erfinder: **Martens, Jürgen, Prof. Dr.**
**Hochstrasse 5**
**D-8755 Alzenau(DE)**

(54) **Verfahren zur Ringspaltung von in 4-Stellung substituierten 2-Isopropyl-5,5-dimtehylthiazolidinen.**

(57) Die Erfindung betrifft ein Verfahren zur Ringspaltung von in 4-Stellung substituierten 2-Isopropyl-5,5-dimethylthiazolidinen in wässrigem, saurem Medium bei erhöhter Temperatur.

Ist der Fünfring in 4-Stellung durch eine Carboxylgruppe substituiert, entsteht Penicillamin.

EP 0 235 376 A2

## Verfahren zur Ringspaltung von in 4-Stellung substituierten 2-Isopropyl-5,5-dimethylthiazolidinen

Die Erfindung betrifft ein Verfahren zur Ringspaltung von 2-Isopropyl-5,5-dimethylthiazolidinverbindungen zu Penicillamin bzw. dessen Derivaten oder Salzen.

Während in 2-Stellung disubstituierte Thiazolidin-4-carbonsäuren bzw. deren Alkylester, Amide oder Salze durch hydrolytische Ringspaltung, zum Beispiel durch kurzzeitiges Erhitzen mit Wasser, rasch in Penicillamin bzw. dessen Derivate oder Salze übergeführt werden können, sind die in 2-Stellung monosubstituierten Thiazolidin-4-carbonsäuren bzw. deren Derivate oder Salze relativ beständig gegen hydrolytische Ringspaltung (Zeitschrift für Naturforschung 18 b, 25, 1963).

Es ist bekannt, 2-Isopropyl-5,5-dimethyl-thiazolidin-4-carbonsäure mit Hilfe von Wasserdampf unter azeotroper Entfernung des sich bildenden Isobutyraldehyds aus dem Gleichgewichtssystem zu Penicillamin zu spalten. Zur quantitativen Spaltung der 2-Isopropyl-5,5-dimethyl-thiaolidin-4-carbonsäure benötigt man jedoch große Mengen Wasserdampf und verhältnismäßig lange Reaktionszeiten. Zur quantitativen Spaltung von 1 Mol 2-Isopropyl-5,5-dimethyl-thiazolidin-4-carbonsäure sind z. B. 20 bis 30 l Wasser zu verdampfen - (DE-PS 1 795 297).

Es besteht auch die Möglichkeit, 2-Isopropyl-5,5-dimethyl-thiazolidinverbindungen mit Hilfe von Hydroxylamin zu spalten (DE-OS 2 142 336).

Bekannt ist auch, daß Penicillamin aus 2,2,5,5-Tetramethyl-thiazolidin-4-carbonitril erzeugt werden kann, indem dieses Nitril entweder unmittelbar oder über die Zwischenstufe des Carbonamids in einen Tetramethyl-thiazolidin-4-carbonsäureester übergeführt, dieser Ester mit Chlorwasserstoffsäure zum Penicillamin-hydrochlorid umgesetzt und das Penicillamin-hydrochlorid mit Alkali neutralisiert wird (Jahrbuch 1967 des Landesamtes für Forschung Nordrhein-Westfalen, 11 bis 35). Dieses Verfahren ist, da die Carbonsäureester als Zwischenprodukt erzeugt werden müssen, aufwendig und ergibt überdies nur geringe Ausbeuten. Die unmittelbare Überführung des Tetramethyl-thiazolidin-4-carbonitrils in die Salze des Penicillamins ist nicht gelungen.

Gegenstand der Erfindung ist ein Verfahren zur Ringspaltung von in 4-Stellung substituierten 2-Isopropyl-5,5-dimethylthiazolidinverbindungen oder deren mineralsauren Salzen durch Hydrolyse in wässrigem saurem Medium, das dadurch gekennzeichnet ist, daß man von einer Verbindung der Formel

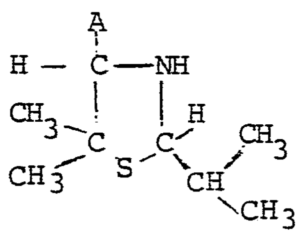

(I) oder deren mineralsauren Salzen ausgeht

in der A für eine, gegebenenfalls durch niedere Alkylgruppen mit 1 bis 6 Kohlenstoffatomen oder durch einen Phenylrest, N-mono-oder N-disubstituierte Carbonamid-oder Thiocarbonsäureamidgruppe steht oder eine COOH-gruppe ist, und die Hydrolyse bei einer Temperatur von 80 bis 220 °C unter dem sich in einem Autoklaven einstellenden Eigendruck durchführt.

Gegebenenfalls arbeitet man unter Inertgasatomsphäre. Anschließend extrahiert man gegebenenfalls mit einem inerten organischen Lösungsmittel und isoliert aus der wässrigen Phase die gewünschten Hydrolyseprodukte bzw. deren Salze in an sich bekannter Weise und spaltet gegebenenfalls das Razemat auf.

Bei dem erfindungsgemäßen Verfahren ist es nicht notwendig, von den reinen Ausgangsstoffen der allgemeinen Formel I auszugehen. Es können auch Gemische mit anorganischen Salzen, zum Beispiel Ammoniumsalzen, wie sie zum Beispiel bei der Hydrolyse des 2-Isopropyl-5,5-dimethylthiazolidin-4-nitrils zur Carbonsäure zwangsläufig anfallen, eingesetzt werden.

Zu den mineralsauren Salzen der 2-Isopropyl-5,5-dimethylthiazolidinverbindungen gemäß Formel I gehören die Hydrohalogenide und insbesondere das Hydrochlorid.

Bevorzugt eingesetzt werden Verbindungen der Formel I, in denen A für die Carboxylgruppe steht und Penicillamin bzw. dessen Salz als gewünschtes Hydrolyseprodukt anfällt.

Verbindungen gemäß Formel I werden bevorzugt in einer möglichst hohen Konzentration eingesetzt, deren konkreter Wert von der jeweiligen Löslichkeit in der Reaktionslösung abhängt.

Als saures Medium werden wässrige Lösungen bevorzugt starker Säuren wie Halogenwasserstoffsäure, Schwefel-und Phosophorsäure verwendet. Es ist vorteilhaft, mit höheren Säurekonzentrationen zu arbeiten, z. B. bei Schwefelsäure mit mehr als 50 Gew.%, bei Salzsäure mit mehr als 20 Gew.%, bevorzugt mehr als 30 Gew.%. Die Ringspaltung mit Hilfe starker Säuren führt man bei Temperaturen durch, die zwischen 80 und 220 °C liegen. Die Reaktionstemperatur ist dabei abhängig von der verwendeten Säure und deren Konzentration. Die Reaktionszeiten sind abhängig von der Temperatur, der Säure und deren Konzentration.

Bei der Durchführung des erfindungsgemäßen Verfahrens geht man zweckmäßigerweise so vor, daß man die 2-Isopropyl-5,5-dimethylthiazolidin-Verbindung in die wässrige Lösung der starken Säure einträgt und dann gegebenenfalls unter Rühren und gegebenenfalls unter Inertgasatmosphäre auf die Reaktionstemperatur erhitzt. Es empfiehlt sich, eine bestimmte Reaktionszeit einzuhalten, da bei Überschreitung einer optimalen Reaktionszeit die Konzentration des gewünschten Spaltproduktes (Penicillamin bzw. dessen Derivate oder Salze) wieder abnimmt. Aus der Reaktionslösung können das Penicillamin bzw. dessen Derivate oder Salze, gegebenenfalls nach Ausschütteln mit einem inerten organischen Lösungsmittel in an sich bekannter Weise, zum Beispiel durch Abdampfen zur Trockne, Abtrennung der anorganischen Salze durch Lösungsmittelextraktion, gewonnen werden (DE-PS 1 795 297). Das Verfahren kann sowohl diskontinuierlich als auch kontinuierlich, zum Beispiel unter Verwendung von Extraktionskolonnen, durchgeführt werden.

Das erhaltene DL-Penicillamin kann nach an sich bekannten Methoden, beispielsweise nach der Brucin-Methode, in die optischen Antipoden aufgespalten werden. Hierbei ist es beispielsweise vorteilhaft, das Penicillamin zunächst in eine Verbindung zu überführen, die für die Razematspaltung besonders geeignet ist. Insbesondere kommt hierfür die Überführung in ein Acylderivat des Penicillamins oder der 2,2-disubstituierten-5,5-dimethyl-thiazolidin-4-carbonsäuren in Frage.

Beispiel 1

5,76 g (24 mmol) 2-Isopropyl-5,5-dimethyl-thiazolidin-4-carbonsäure . HCl werden in 60 ml 12 n Salzsäure eingetragen und in einem Autoklaven ca. 105 min. auf 170 °C erhitzt. Nach Abkühlen auf Raumtemperatur wird durch hochdruckflüssigkeits-chromatographische Analyse ein Gehalt von 19,5 mmol - (81,3 % d. Th.) Penicillamin . HCl festgestellt. Die Hauptmenge der Salzsäure wird nun abdestilliert, bis eine Sumpftemperatur von ca. 125 °C erreicht ist. Nach Zugabe von 100 ml Toluol zur Sumpfflüssigkeit wird der Rest Salzsäure bzw. Wasser azeotrop abdestilliert, bis eine Sumpftemperatur von ca. 99 °C erreicht ist. Nach dem Abkühlen werden 40 ml Aceton zugegeben und die Mischung auf eine Temperatur von 70 -80 °C gebracht. Nach kurzer Zeit fällt 2,2,5,5-Tetramethyl-thiazolidin-4-carbonsäure . HCl aus und wird nach 3 Stunden abfiltriert und getrocknet. Man erhält 4,0 g (73,8 % d.Th) reines 2,2,5,5-Tetramethyl-thiazolidin-4-carbonsäure . HCl vom Schmp. 206 -208 °C.

Beispiel 2

Es wird, wie in Beispiel 1 beschrieben, verfahren, jedoch geht man von 11,52 g (48 mmol) 2-Isopropyl-5,5-dimethyl-thiazolidin-4-carbonsäure . HCl in 60 ml 12 n Salzsäure aus und die Reaktionszeit beträgt ca. 120 min. Durch hochdruckflüssigkeits-chromatographische Analyse findet man 37,5 mmol (78,1 % d.Th.) Penicillamin . HCl. Bei der analogen Aufarbeitung erhält man 7,96 g (73,4 % d.Th.) reines 2,2,5,5-Tetramethyl-thiazolidin-4-carbonsäure . HCl vom Schmp. 206 -208 °C.

Beispiel 3

11,52 g (48 mmol) 2-Isopropyl-5,5-dimethyl-thiazolidin-4-carbonsäure . HCl werden in 60 ml 12 n Salzsäure eingetragen und in einem Autoklaven ca. 120 min auf 170 °C erhitzt. Nach Abkühlen auf Raumtemperatur findet man mittels hochdruckflüssigkeits-chromatographischer Analyse einen Gehalt von 39 mmol (81,3 % d.Th.) Penicillamin . HCl. Die Lösung wird nun einmal mit 10 ml Chloroform ausgeschüttelt und die wässrige Phase am Rotationsverdampfer zur Trockne eingeengt. Den Rückstand löst man

in 70 ml 96%igem Äthanol und stellt die Lösung mit Hilfe von Triäthylamin in Äthanol auf einen pH-Wert zwischen 6 und 7 ein. Nach kurzer Zeit scheidet sich Penicillamin ab. Nach Trocknung erhält man 5,46 g - (76,3 % d.Th) Penicillamin vom Schmp. 201 -204 °C.

Beispiel 4

Eine 0,8 molare Lösung (0,8 mol/l) von 2-Isopropyl-5,5-dimethyl-thiazolidin-4-carbonsäure . HCl in 12 n Salzsäure wird in einem Autoklaven erhitzt. Die Reaktionszeit und die Reaktionstemperatur werden variiert. Nach dem Abkühlen bestimmt man die Ausbeute an Penicillamin . HCl durch hochdruckflüssigkeits-chromatographische Analyse. Die Ergebnisse sind in der Tab. 1 zusammengefasst:

Tabelle 1

| Reaktionstemperatur (°C) | Reaktionszeit (min) | Ausbeute (%) |
|---|---|---|
| 150 | 160 | 38,1 |
| | 250 | 59,4 |
| | 340 | 69,4 |
| | 490 | 63,8 |
| 170 | 105 | 78,1 |
| | 135 | 83,1 |
| | 165 | 69,4 |
| | 195 | 46,9 |
| 190 | 60 | 68,8 |
| | 75 | 64,6 |
| | 90 | 43,8 |

Beispiel 5

Eine 0,8 molare (o,8 mol/l) Lösung von 2-Isopropyl-5,5-dimethyl-thiazolidin-4-carbonsäure . HCl in 47%iger Bromwasserstoffsäure wird in einem Autoklaven auf 170 °C erhitzt. Die Reaktionszeit wird variiert. Nach dem Abkühlen bestimmt man die Ausbeute an Penicillamin . HCl durch hochdruckflüssigkeits-chromatographische Analyse. Die Ergebnisse sind in der Tab. 2 zusammengefasst:

Tabelle 2

| Reaktionszeit (min) | Ausbeute (%) |
|---|---|
| 75 | 75 |
| 100 | 77,1 |
| 120 | 70,8 |
| 140 | 71,9 |

Beispiel 6

Eine 0,8 molare (0,8 mol/l) Lösung von 2-Isopropyl-5,5-dimethyl-thiazolidin-4-carbonsäure . HCl in 75%iger Schwefelsäure wird in einem Autoklaven erhitzt. Die Reaktionszeit und die Reaktionstemperatur werden variiert. Nach dem Abkühlen bestimmt man die Ausbeute an Penicillamin . HCl durch hochdruckflüssigkeits-chromatographische Analyse. Die Ergebnisse sind in der Tabelle 3 zusammengefasst:

Tabelle 3

| Reaktionstemperatur (°C) | Reaktionszeit (min) | Ausbeute (%) |
|---|---|---|
| 130 | 75 | 25,0 |
| | 135 | 43,8 |
| | 195 | 45,0 |
| 140 | 45 | 33,3 |
| | 75 | 55,8 |
| | 105 | 50,0 |
| 160 | 30 | 35,0 |
| | 45 | 33,3 |
| | 60 | 25,0 |

**Ansprüche**

1. Verfahren zur Ringspaltung von in 4-Stellung substituierten 2-Isopropyl-5,5-dimethylthiazolidinverbindungen oder deren mineralsauren Salzen durch Hydrolyse in wässrigem saurem Medium, dadurch gekennzeichnet, daß man von einer Verbindung der Formel

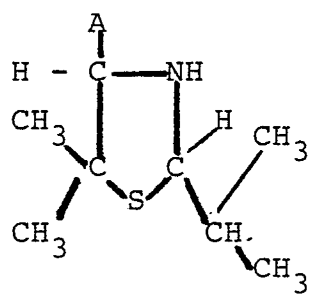

(I) oder deren mineralsauren Salzen ausgeht

in der A für eine, gegebenenfalls durch niedere Alkylgruppen mit 1 bis 6 Kohlenstoffatomen oder durch einen Phenylrest, N-mono-oder N-disubstituierte Carbonamid-oder Thiocarbonsäureamidgruppe steht oder eine COOH-Gruppe ist, und die Hydrolyse bei einer Temperatur von 80 bis 220 °C unter dem sich in einem Autoklaven einstellenden Eigendruck durchführt.

2. Verfahren nach Anspruch 1, dadurch gekennzeichnet, daß A für eine COOH-Gruppe steht und durch die Hydrolyse Penicillamin entsteht.

3. Verfahren nach den Ansprüchen 1 und 2, dadurch gekennzeichnet, daß man in wässriger Schwefelsäurelösung mit einer Konzentration von > 50 Gew.% Schwefelsäure arbeitet.

4. Verfahren nach den Ansprüchen 1 und 2, dadurch gekennzeichnet, daß man in einer Salzsäurelösung mit > 20 Gew.% Salzsäure arbeitet.